Europäisches Patentamt
European Patent Office
Office européen des brevets

(11) Veröffentlichungsnummer: **0 000 506**
**A2**

(12) # EUROPÄISCHE PATENTANMELDUNG

(21) Anmeldenummer: 78100374.4

(22) Anmeldetag: 12.07.78

(51) Int. Cl.²: C 07 C 43/26, C 07 C 43/28, C 07 C 41/00, C 07 C 149/36, C 07 C 148/00, C 07 C 69/74, A 61 K 31/08, // C07C49/28, C07C49/30, C07C69/16, C07C43/27

(30) Priorität: 20.07.77 CH 9002/77

(43) Veröffentlichungstag der Anmeldung: 07.02.79 Bulletin 79/3

(84) Benannte Vertragsstaaten: BE CH DE FR GB LU NL SE

(71) Anmelder: CIBA-GEIGY AG
Patentabteilung Postfach
CH-4002 Basel. (CH)

(72) Erfinder: Farooq, Saleem, Dr.
Im Schalengarten
CH-4107 Ettingen. (CH)

(72) Erfinder: Karrer, Friedrich, Dr.
Rebbergstrasse 5
CH-4800 Zofingen. (CH)

(72) Erfinder: Haas, Georges, Dr.
Im Rehwechsel 24
CH-4102 Binningen. (CH)

(54) **Mit substituierten Phenyl O-oder S- verätherte Cycloalkanole, Verfahren zu deren Herstellung und deren Anwendung als Hypolipidaemika; deren Cyclopropan-carbonsäureester und Anwendung dieser Ester als Insektizide.**

(57) Verätherte Cycloalkanole der Formel

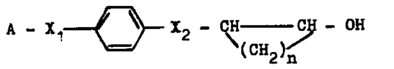

worin $X_1$ Sauerstoff, Schwefel oder Methylen, $X_2$ Sauerstoff oder Schwefel, A einen gegebenenfalls substituierten Phenylrest und n eine ganze Zahl von 1 bis und mit 10 darstellen sowie Verfahren zu deren Herstellung.

Diese Verbindungen, insbesondere das 2-(4-Phenoxyphenoxy)-cyclopentan-1-ol, besitzen eine Lipidsenkende Wirksamkeit.

Ferner sind sie Zwischenprodukte zur Herstellung der insektiziden Cyclopropansäureester der Formel

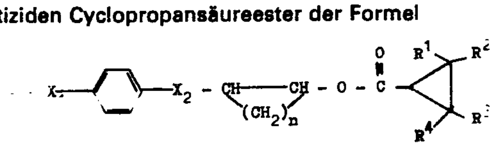

worin A, $X_1$, $X_2$, und n die oben gegebene Bedeutung besitzen und $R^1$, $R^2$, $R^3$ und $R^4$ je Wasserstoff, Methyl oder Chlor bedeuten.

EP 0 000 506 A2

4-11262/+

BEZEICHNUNG GEÄNDERT
siehe Titelseite

Verätherte Cycloalkanole und ein Verfahren zu deren Herstellung

Die Erfindung betrifft neue verätherte Cycloalkanole, insbesondere solche der Formel (I)

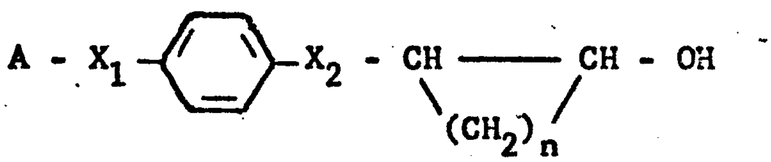

(I)

worin $X_1$ Sauerstoff, Schwefel oder Methylen, $X_2$ Sauerstoff oder Schwefel, A einen gegebenenfalls substituierten Phenylrest und n eine ganze Zahl von 1 bis und mit 10 darstellen, sowie Verfahren zu deren Herstellung, wie auch pharmazeutische Präparate enthaltend diese Verbindungen und deren Verwendung vorzugsweise in Form von pharmazeutischen Präparaten.

Die im Zusammenhang mit der vorliegenden Beschreibung mit "nieder" bezeichneten Reste und Verbindungen enthalten vorzugsweise bis und mit 7 und in erster Linie bis und mit 4 Kohlenstoffatome.

Ein substituierter Phenylrest A ist ein mono-, di- oder polysubstituierter Phenylrest. Als Substituenten seien insbesondere genannt: Halogen bis einschliesslich Atomnummer 35, Niederalkyl, Niederalkoxy oder Trifluormethyl.

Die Allgemeinbegriffe können folgende Bedeutung haben:

Niederalkyl ist z.B. n-Propyl, Isopropyl, n-Butyl, Isobutyl, sek.-Butyl, tert.-Butyl, n-Pentyl, Isopentyl, n-Hexyl, Isohexyl oder n-Heptyl, aber vor allem Methyl oder Aethyl.

Niederalkoxy ist z.B. n-Propoxy, Isopropoxy oder n-Butyloxy, besonders aber Methoxy oder Aethoxy.

Halogen steht für Fluor oder Brom, vorzugsweise jedoch für Chlor.

Vorzugsweise stehen $X_2$ und die Hydroxygruppe in trans-Stellung zu einander.

Die neuen Verbindungen besitzen wertvolle pharmakologische Eigenschaften. So bewirken sie eine Senkung des Lipidgehaltes im Serum, wie das z.B. an der männlichen Ratte, welche während 3 Tagen jeweils eine Dosis von 10 bis 200 mg/kg der Testsubstanzen und am 4 Tage zwei solche Dosen erhält und anschliessend Bestimmung der Cholesterin- und Triglycerid-Konzentration im Serum, gezeigt werden kann. Sie können somit als Hypolipidaemika zur Behandlung von Lipidstoffwechselstörungen verwendet werden.

Sie können aber auch als Zwischenprodukte dienen, insbesondere zur Herstellung von Cyclopropancarbonsäureestern, welche sich zur Bekämpfung von verschiedenartigen tierischen und pflanzlichen Schädlingen, insbesondere zur Bekämpfung von Vertretern der Ordnung Akarina der Familien: Ixodidae, Argasidae, Tetranychidae, Dermanyssidae, sowie von Insekten der Familien: Acrididae, Blattidae, Gryllidae, Gryllotalpidae, Tettigoniidae, Cimicidae, Pyrrhocoridae, Reduviidae, Aphididae, Delphacidae, Diaphididae, Pseudococcidae, Chrysomilidae, Coccinellidae, Bruchidae, Scarabaidae, Dermestidae, Tenebrionidae, Curculionidae, Tincidae, Noctuidae, Lymantriidae, Pyralidae, Galleridae, Culicidae, Tipulidae, Stomoxydae, Muscidae, Calliphoridae, Trypertidae oder Pulicidae.

Die Erfindung betrifft insbesondere Verbindungen der Formel (II)

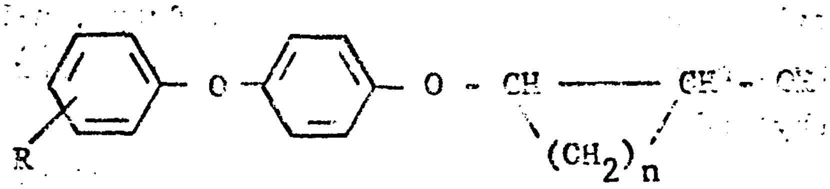

worin R Wasserstoff oder Chlor und n die ganzen Zahlen 3, 4 oder 5 darstellen.

In allererster Linie betrifft die Erfindung das 1-(4-Phenoxy-phenoxy)-cyclopentan-2-ol.

Die Erfindung betrifft auch vorzugsweise die Verbindungen der Formel

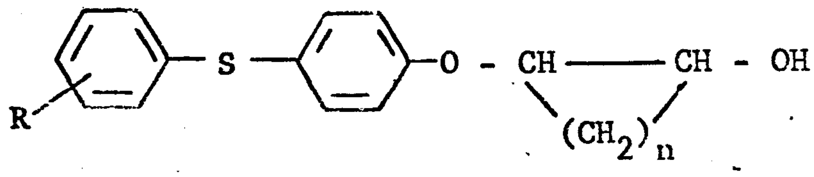

(III)

worin R Wasserstoff oder Chlor und n die ganzen Zahlen 3, 4 oder 5 darstellen oder auch die Verbindungen der Formel

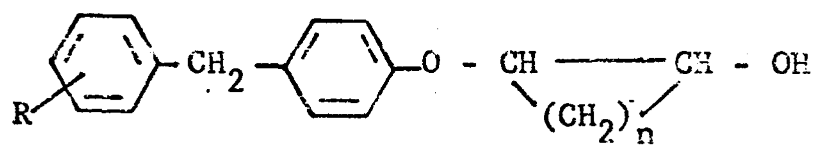

(IIIa)

worin R Wasserstoff oder Chlor und n die ganzen Zahlen 3, 4 oder 5 darstellen.

Die Erfindung betrifft ganz besonders die in den Beispielen beschriebenen, neuen Verbindungen.

Die neuen Verbindungen werden in an sich bekannter Weise erhalten; so kann man z.B. eine Verbindung der Formel (IV)

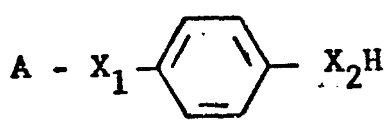

(IV)

oder ein Salz davon, mit einer Verbindung der Formel (V)

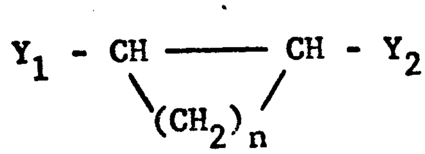

(V)

umsetzen, worin $Y_1$ eine freie oder reaktionsfähig veresterte Hydroxygruppe und $Y_2$ eine gegebenenfalls geschützte Hydroxylgruppe oder $Y_1$ und $Y_2$ zusammen eine Epoxygruppe

BAD ORIGINAL

oder eine Carbonyldioxygruppe der Formel -OC(O)O- darstellen, und in erhaltenen Verbindungen eine gegebenenfalls vorhandene Schutzgruppe abspalten, und, wenn erwünscht, ein verfahrensgemäss erhältliches Isomerengemisch in die einzelnen Isomeren auftrennen.

Salze von Verbindungen der Formel (IV) sind in erster Linie Metall-, insbesondere Alkalimetall- z.B. Natrium- oder Kaliumsalze, die üblicherweise in situ hergestellt werden.

Eine reaktionsfähig veresterte Hydroxygruppe ist insbesondere eine solche die mit einer starken Säure, wie einer starken anorganischen oder organischen Säure verestert ist. Als starke anorganische Säuren seien insbesondere Halogenwasserstoffsäuren, wie Chlor- oder Bromwasserstoffsäure, oder Schwefelsäure genannt und als organische Säuren, in erster Linie Sulfonsäuren, wie eine gegebenenfalls durch Halogen, Niederalkyl oder Niederalkoxy substituierte Benzolsulfonsäure, z.B. p-Toluolsulfonsäure oder p-Methoxybenzolsulfonsäure, oder eine Alkansulfonsäure, z.B. Methan- oder Aethansulfonsäure.

Eine geschützte Hydroxygruppe ist insbesondere eine veresterte Hydroxygruppe, z.B. eine Acyloxygruppe, wie eine Niederalkanoyloxy-, z.B. Acetoxy- oder eine Benzoyloxygruppe oder eine verätherte Hydroxygruppe, z.B. eine Tetrahydropyranyloxy- oder eine Aralkoxygruppe, wie die Benzyloxygruppe.

Die Reaktion wird in an sich bekannter Weise vorgenommen. Dabei arbeitet man vorzugsweise in Gegenwart einer Base, insbesondere einer anorganischen Base, wie einem Alkali- oder Erdalkali-hydroxyd, -carbonat oder -hydrid, z.B. Natrium- oder Kaliumhydroxyd, -hydrid oder -carbonat oder einer organischen Base, wie Piperidin, Pyridin, Chinolin oder einem Triniederalkylamin, z.B. Trimethylamin, Triäthylamin oder Dimethylisopropylamin. Man kann sie aber

auch in Gegenwart eines Carbodiimids, wie Dicyclohexylcarbodiimid in Gegenwart von Kupferchlorid, durchführen. Dabei kann in einem inerten Lösungsmittel, wie Aceton oder Dimethylsulfoxyd arbeiten. Die Reaktion kann aber auch in einem höher siedenden Lösungsmittel, wie Xylol, Toluol oder Chinolin bei erhöhter Temperatur, z.B. 80-200°, oder in Abwesenheit eines Lösungsmittels erfolgen.

Eine geschützte Hydroxygruppe lässt sich in an sich bekannter Weise, z.B. durch Hydrolyse oder Hydrogenolyse in Freiheit setzen.

Die zu dieser Reaktion zu verwendenden Ausgangsstoffe sind bekannt, oder lassen sich auf an sich bekannte Weise erhalten.

Die neuen Verbindungen lassen sich aber auch erhalten, wenn man in einer Verbindung der Formel (VI)

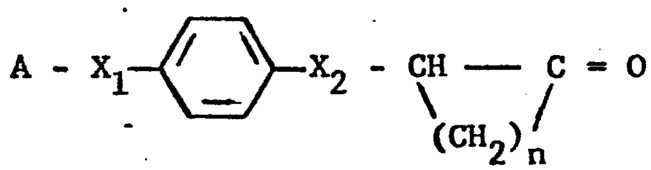

(VI)

die Ketogruppe in an sich bekannter Weise zur Carbinolgruppe reduziert, und, wenn erwünscht, ein verfahrensgemäss erhältliches Isomerengemisch in die einzelnen Isomeren auftrennt.

Die Reduktion lässt sich in üblicher Weise z.B. mit katalytisch aktiviertem Wasserstoff, z.B. in Gegenwart von Raney-Nickel oder eines Palladiumkatalysators wie Pd/C oder mit Metallhydriden, wie Natriumborhydrid, Lithiumaluminiumhydrid oder Diboran, in Gegenwart eines inerten Lösungsmittels, wie Wasser, Alkoholen, z.B. Methanol oder Aethanol, oder Aethern, wie Diäthyläther oder Tetrahydrofuran durchführen.

Das in dieser Verfahrensmethode verwendete Ausgangsmaterial wird in an sich bekannter Weise erhalten, z.B. durch Umsetzen eines Keto-cycloalkanols oder eines reaktionsfähigen Esters davon mit einer Verbindung der Formel IV in der oben gezeigten Art, oder durch Umsetzen einer Verbindung der Formel IV mit einem reaktionsfähigen Ester eines 3-Hydroxy-cycloalkens und anschliessender Oxydation der Doppelbindung, z.B. mit Quecksilber-II-nitrat oder acetat in Wasser, oder einer Persäure wie Peressigsäure. Das so erhaltene Ausgangsmaterial der Formel (VI) lässt sich, wenn erwünscht, ohne Isolierung reduzieren.

Die neuen Verbindungen können als Isomerengemische, wie Racemate oder Diastereoisomerengemische, oder in Form der reinen Isomeren, wie der optisch aktiven Komponenten, vorliegen. Die Auftrennung von erhaltenen Isomerengemischen in die individuellen Isomeren kann nach bekannten Methoden geschehen. Diastereoisomerengemische lassen sich z.B. auf Grund physikalisch-chemischer Unterschiede, wie solchen der Löslichkeit, z.B. durch fraktioniertes Kristallisieren oder Destillieren, oder durch Chromatographie in die einzelnen Isomeren auftrennen.

Racemate kann man z.B. durch Verestern des Alkohol-racemates mit einer optisch aktiven Säure, z.B. optisch aktiver Camphersulfonsäure, Trennen des so erhältlichen Ester-Diastereoisomerengemisches und Verseifen des Esters oder durch Verestern des Alkohol-racemates mit einer Dicarbonsäure, wobei nur eine Carboxylgruppe verestert wird, Bilden eines Salzes des so erhältlichen Monoester-säureracemates mit einer geeigneten optisch aktiven Base, z.B. optisch aktives Brucin, α-Phenyläthylamin oder Ephedrin, Aufspalten des so erhältlichen Salz-Diastereoisomerenge-

mischen und Verseifen des erhältlichen Estersalzes, in die optisch aktiven Antipoden auftrennen. Dabei isoliert man vorteilhafterweise dasjenige Isomere, das die pharmakologisch wertvolleren Eigenschaften aufweist.

Die oben beschriebenen Verfahren werden nach an sich bekannten Methoden durchgeführt, in Abwesenheit oder vorzugsweise in Anwesenheit von Verdünnungs- oder Lösungsmitteln, wenn notwendig, unter Kühlen oder Erwärmen, unter erhöhtem Druck und/oder in einer Inertgas-, wie Stickstoffatmosphäre.

Die Erfindung betrifft auch diejenigen Ausführungsformen des Verfahrens, bei denen man von einer auf irgendeiner Stufe des Verfahrens als Zwischenprodukt erhältlichen Verbindung ausgeht und die fehlenden Verfahrensschritte durchführt, oder das Verfahren auf irgendeiner Stufe abbricht, oder einen Ausgangsstoff unter den Reaktionsbedingungen bildet oder in Form eines reaktionsfähigen Derivats oder Salzes verwendet. Dabei geht man vorzugsweise von solchen Ausgangsstoffen aus, die verfahrensgemäss zu den oben als besonders wertvoll beschriebenen Verbindungen führen.

Die vorliegende Erfindung betrifft ebenfalls pharmazeutische Präparate, welche Verbindungen der Formel I enthalten. Bei den erfindungsgemässen pharmazeutischen Präparaten handelt es sich um solche zur enteralen, wie oralen oder rektalen, sowie parenteralen Verabreichung an Warmblüter, welche den pharmakologischen Wirkstoff allein oder zusammen mit einem pharmazeutisch anwendbaren Trägermaterial enthalten. Die Dosierung des Wirkstoffes hängt von der Warmblüter-Spezies, dem Alter und dem individuellen Zustand, sowie von der Applikationsweise ab.

Die pharmazeutischen Präparate der vorliegenden Erfindung können z.B. in Dosiseinheiten, wie Dragées, Tabletten, Kapseln, Suppositorien oder Ampullen vorliegen und werden in an sich bekannter Weise, z.B. mittels konventioneller Misch-, Granulier-, Dragier-, Lösungs- oder Lyophilisierungsverfahren hergestellt. Pharmazeutische Präparate insbesondere zur oralen Anwendung können erhalten werden, indem man den Wirkstoff mit festen Trägerstoffen kombiniert, ein erhaltenes Gemisch gegebenenfalls granuliert, und das Gemisch bzw. Granulat, wenn erwünscht oder notwendig nach Zugabe von geeigneten Hilfsstoffen, zu Tabletten oder Dragée-Kernen verarbeitet.

Geeignete Trägerstoffen sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose, Hydroxypropyl-methylcellulose, Natriumcarboxymethylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrodidon, Polyäthylenglycol und/oder Titandioxid enthalten, Lacklösungen in geeigneten organi-

BAD ORIGINAL

schen Lösungsmitteln oder Lösungsmittelgemischen oder Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Die pharmazeutischen Präparate enthalten von etwa 0,1 % bis 100%, inbesondere von etwa 1% bis etwa 50 % des Aktivstoffes. Die Einzeldosis für einen Warmblüter von etwa 70 kg Gewicht beträgt zwischen 0,1 und 0,75 g, die Tagesdosis zwischen 0,2 und 1 g

Die Erfindung betrifft auch neue Cyclopropancarbonsäureester der Formel (VII)

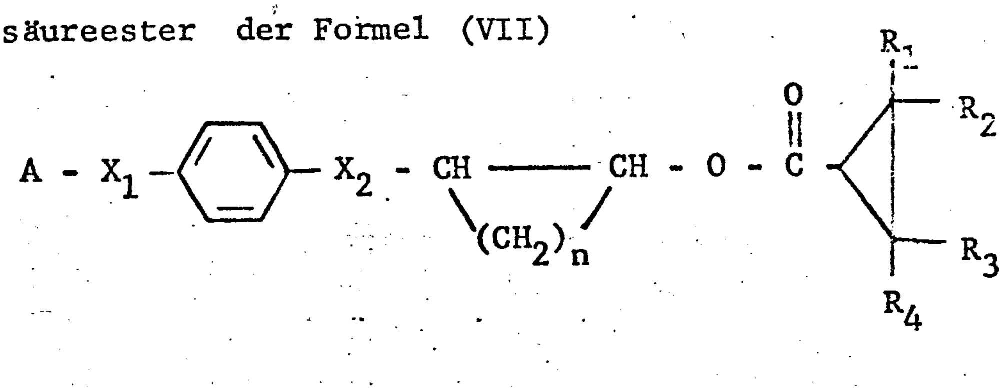

(VII)

worin A, $X_1$, $X_2$ und n die oben gegebene Bedeutung besitzen und $R_1$, $R_2$, $R_3$ und $R_4$ je Wasserstoff, Methyl oder Chlor bedeuten, deren Herstellung und ihre Verwendung in der Schädlingsbekämpfung.

Wegen ihrer Wirkung bevorzugt man Verbindungen der Formel VII, worin A Phenyl, $X_1$ und $X_2$ Sauerstoff bedeuten. Weiterhin bevorzugt sind Verbindungen der Formel VII, worin $R_1$, $R_2$, $R_3$ und $R_4$ entweder Wasserstoff oder Methyl bedeuten. Hervorzuheben sind ferner Verbindung der Formel VII, worin $R_1$ und $R_2$ Chlor und $R_3$ und $R_4$ Methyl bedeuten.

BAD ORIGINAL

Wie oben bereits bemerkt eignen sich diese Ester ausgezeichnet zur Bekämpfung von verschiedenartigen tierischen und pflanzlichen Schädlingen.

Die Verbindungen der Formel VII werden nach an sich bekannten Methoden, z.B. wie folgt hergestellt:

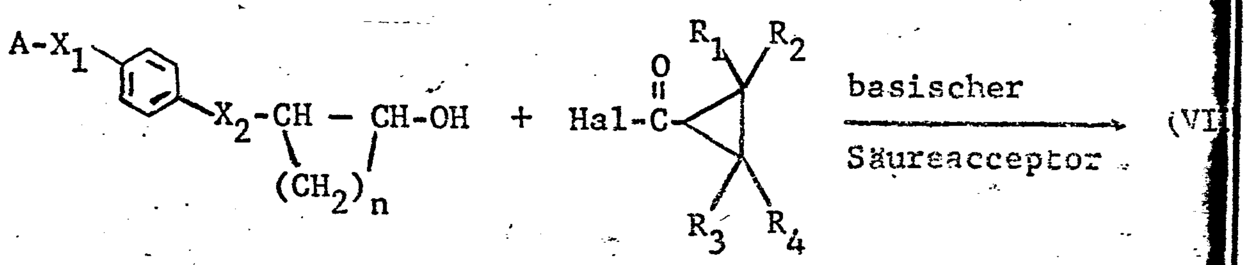


(VIII) (IX)

Hal steht darin für Halogen, insbesondere für Chlor oder Brom.

Als basische Säureacceptoren kommen z.B. in Frage: tertiäre Amine, wie Trialkylamine, z.B. Aethyl-diisopropylamin; Pyridin; Dialkylaniline; ferner anorganische Basen, wie Hydride, Hydroxide, Alkoxide und Karbonate von Alkali- und Erdalkalimetallen.

Das Verfahren wird im allgemeinen bei einer Temperatur von etwa 10°C bis 100°C, mit Säurehalogeniden meist bei 0°C bis 30°C und mit Säureanhydriden meist bei 70°C bis 100°C, bei normalem Druck und in einem inerten Lösungs- oder Verdünnungsmittel durchgeführt. Als Lösungs- oder Verdünnungsmittel eignen sich z.B. Benzol, Toluol, Xylol; Paraffinkohlenwasserstoffe wie Hexan oder Heptan; Aether wie Diäthyläther, Tetrahydrofuran, Dioxan, Dimethoxyäthan; ferner Ester, wie Essigsäureäthylester.

Die insektizide Wirkung lässt sich durch Zusatz von anderen Insektiziden und/oder Akariziden wesentlich verbreitern und an gegebene Umstände anpassen.

Als Zusätze eignen sich z.B.:
- organische Phosphorverbindungen,
- Nitrophenole und Derivate,
- Formamidine, Harnstoffe, Carbamate,
- Chrysanthemumsäure-Derivate oder
- chlorierte Kohlenwasserstoffreste.

Die Verbindungen der Formel VII können für sich allein oder zusammen mit geeigneten Trägern und/oder Zuschlagstoffen eingesetzt werden. Geeignete Träger und Zuschlagstoffe können fest oder flüssig sein und entsprechen den in der Formulierungstechnik üblichen Stoffen, wie z.B. natürlichen oder regenerierten Stoffe, Lösungs-, Dispergier-, Netz-, Haft-, Verdickungs-, Binde- und/oder Düngemitteln. Zur Applikation können die Verbindungen der Formel VII mit Stäubemitteln, Emulsionskonzentraten, Granulate, Disperionen, Sprays, zu Lösungen oder Aufschlämmungen in üblicher Formulierung, die in der Applikationstechnik zum Allgemeinwissen gehören, verarbeitet werden.

Die Herstellung erfindungsgemässer Mittel erfolgt in an sich bekannter Weise durch inniges Vermischen und/oder Vermahlen von Wirkstoffen der Formel VII mit den geeigneten Trägerstoffen, gegebenenfalls unter Zusatz von gegenüber den Wirkstoffen inerten Dispergier- oder Lösungsmitteln. Die Wirkstoffe können in den folgenden Aufarbeitungsformen vorliegen und angewendet werden.

| | |
|---|---|
| Feste Aufarbeitungsformen: | Stäubemittel, Streumittel, Granulate (Umhüllungsgranulate, Imprägnierungsgranulate und Homogengranulate); |

Flüssige Aufarbeitungsformen:

a) in Wasser dispergierbare Wirkstoffkonzentrate: Spritzpulver (wettable powder) Pasten, Emulsionen;

b) Lösungen.

Der Gehalt an Wirkstoffen in den oben beschriebenen Mitteln liegt zwischen 0,1 bis 95 Gew.-%.

Die folgenden Beispiele dienen zur Illustration der Erfindung. Temperaturen sind in Celsiusgraden angegeben.

Beispiel 1

Ein Gemisch von 32,0 g 4-Hydroxy-diphenyläther und 23,5 g 1,2-Epoxycyclopentan wird auf 150° geheizt. Zu dieser Schmelze wird innerhalb 25 Minuten, 6,7 g 90%-iges Kaliumhydroxydpulver zugegeben und das Ganze während einer Stunde bei 130° gerührt. Nach dem Abkühlen nimmt man das Reaktionsgemisch in Aether:Hexan (1:1) auf und wäscht die organische Lösung viermal mit 10%-iger Kaliumhydroxydlösung und dreimal mit gesättigter Kochsalzlösung. Die organische Phase wird über Natriumsulfat getrocknet und am Rotationsvaporisator bei vermindertem Druck eingeengt. Man destilliert das Rohprodukt und erhält das 2-(4-Phenoxyphenoxy)-cyclopentan-1-ol als farbloses Oel vom Siedepunkt $Kp_{0.001}$ 181-185° und dem Brechungsindex $n_D^{20} = 1.5812$.

In analoger Weise erhält man das 2-(4-Phenoxyphenoxy)-cyclohexan-1-ol, das nach dem Umkristallisieren aus Hexan bei 62-65° schmilzt und das 2-(4-Benzylphenoxy)-cyclohexan-1-ol das nach dem Umkristallisieren aus Isopropanol bei 78-80° schmilzt.

Beispiel 2

In analoger Weise zu dem im Beispiel 1 beschriebenen Verfahren erhält man, ausgehend von den entsprechenden Ausgangsstoffen,

das 2-(4-Phenylmercaptophenoxy)-cyclopentan-1-ol als farbloses Oel vom Brechungsindex $n_D^{20} = 1,6217$;

das 2-(4-Benzylphenoxy)-cyclopentan-1-ol als Oel vom Brechungsindex $n_D^{20} = 1,5794$;

das 2-[4-(4-Chlor-phenoxy)-phenoxy]-cyclopentan-1-ol vom F. = 53-55°;

das 2-[4-(4-Chlor-phenoxy)-phenoxy]-cyclohexan-1-ol vom F. 82-83°;
das 2-(4-Phenylmercaptophenoxy)-cyclohexan-1-ol vom F. 79-81°;
das 2-[4-(2,4-Dichlor-phenoxy)-phenoxy]-cyclohexan-1-ol vom F. 84-86°;
das 2-[4-(2,4-Dichlor-phenoxy)-phenoxy]-cyclopentan-1-ol als Oel vom Brechungsindex $n_D^{20}$ = 1,5946;
das 2-[4-(2-Methyl-phenoxy)-phenoxy]-cyclohexan-1-ol vom F. 73-75°;
das 2-[4-(4-Chlor-phenylmercapto)-phenoxy]-cyclohexan-1-ol vom F. 90-92°;
das 2-(4-Phenoxy-phenylmercapto)-cyclohexan-1-ol als Oel vom Brechungsindex $n_D^{20}$ = 1,6051;
das 2-(4-Phenoxy-phenylmercapto)-cyclopentan-1-ol, als Oel vom Brechungsindex $n_D^{20}$ = 1,6101;
das 2-[4-(2-Methyl-phenoxy)-phenoxy]-cyclopentan-1-ol, als Oel vom Brechungsindex $n_D^{20}$ = 1,5757 oder
das 2-[4-(4-Chlor-phenylmercapto)-phenoxy]-cyclopentan-1-ol, als Oel vom Brechungsindex $n_D^{20}$ = 1,6212.

Beispiel 3

Zu einer Lösung von 8,04 g 2-(4-Phenoxyphenoxy)-cyclopentan-1-on in 60 ml Methanol gibt man bei Raumtemperatur eine Lösung von 3,78 g Natriumborhydrid in 10 ml Wasser. Das Reaktionsgemisch wird noch 15 Stunden bei Raumtemperatur gerührt und anschliessend unter vermindertem Druck eingeengt. Man giesst den Rückstand auf Eiswasser und extrahiert mit Aether. Die organische Phase wird zweimal mit gesättigter Kochsalzlösung gewaschen, über Natriumsulfat getrocknet und am Rotationsvaporisator einge-

engt. Fraktionierte Destillation des Rückstandes gibt das 2-(4-Phenoxyphenoxy)-cyclopentan-1-ol als farbloses Oel vom $Kp_{0.001}$ 181-185°.

Das Ausgangsmaterial lässt sich z.B. wie folgt erhalten:

Zu einer Lösung von 18,6 g 4-Phenoxyphenol in 80 ml Aceton werden 15,2 g wasserfreies Kaliumcarbonat gegeben und das Reaktionsgemisch eine Stunde am Rückfluss gekocht. Man tropft dann 17,3 g 2-Brom-cyclopentanon zu und kocht während 20 Stunden am Rückfluss. Man kühlt das Reaktionsgemisch ab, filtriert und engt das Filtrat am Rotationsvaporisator ein. Man nimmt das erhaltene Oel in Aether auf, wäscht die Lösung viermal mit 10%-iger Kaliumhydroxydlösung und zweimal mit gesättigter Kochsalzlösung, trocknet über Natriumsulfat und engt am Rotationsvaporisator ein. Das erhaltene rohe 2-(4-Phenoxyphenoxy)-cyclopentan-1-on wird ohne weitere Reinigung für die oben beschriebene Umsetzung verwendet.

## Beispiel 4

In analoger Weise zum vorstehenden Beispiel 3 erhält man ausgehend von den entsprechenden Ausgangsstoffen das 2-(4-Phenoxyphenoxy)-cyclohexan-1-on vom F. = 103-105°, das wie oben beschrieben zum entsprechenden 2-(4-Phenoxyphenoxy)-cyclohexan-1-ol vom F. = 62-65° reduziert wird.

## Beispiel 5

Zu einer Lösung von 18,6 g 4-Phenoxyphenol in 120 ml Dimethylsulfoxyd werden unter Eiskühlung 6,7 g 90%-iger Kaliumhydroxydpulver zugegeben und das Ganze 1/2 Stunde bei Raumtemperatur gerührt. Man gibt dann 16,3 g 2-Acetoxy-cyclopentylchlorid im Laufe von 20 Minuten zu, rührt

dann das Reaktionsgemisch bei 50° ca. 18. Stunden. Man giesst auf Eiswasser, extrahiert mit Aether, wäscht die organische Phase nochmals mit Wasser und einmal mit gesättigter Kochsalzlösung, trocknet über Natriumsulfat und engt ein. Das erhaltene 1-Acetoxy-2-(4-phenoxyphenoxy)-cyclopentan liegt nach fraktionierter Destillation als farbloses Oel mit einem Brechungsindex von $n_D^{20}$ = 1.5560 vor.

Zu einer Lösung von 6,24 g 1-Acetoxy-2-(4-phenoxyphenoxy)-cyclopentan in 50 ml Aethanol tropft man bei Raumtemperatur eine Lösung von 1,68 g Kaliumhydroxyd gelöst in 20 ml Wasser zu, kocht dann 3 Stunde am Rückfluss und dampft nach dem Abkühlen am Rotationsvaporisator auf 1/3 des ursprünglichen Volumens ein. Man giesst den Rückstand auf Eiswasser, extrahiert mit Aether, wäscht die organische Phase dreimal mit gesättigter Kochsalzlösung, trocknet über Natriumsulfat und engt am Rotationsvaporisator ein. Destillation des Rückstandes gibt das 2-(4-Phenoxyphenoxy)-cyclopentan-1-ol als farbloses Oel vom $Kp_{0.001}$ 181-185°.

Beispiel 6

Zu einem Gemisch von 15.9 g Quecksilber(II)acetat, 35 ml Wasser und 50 ml Tetrahydrofuran tropft man im Laufe einer Stunde eine Lösung von 12,6 g 3-(4-Phenoxyphenoxy)-cyclopent-1-en in 25 ml Tetrahydrofuran zu und rührt ca. 15 Stunden bei Raumtemperatur. Dann tropft man 50 ml 3N-Natronlauge innerhalb 1 1/2 Stunden zu und anschliessend innerhalb 1 Stunde 50 ml einer 0,5N-Lösung von Natriumborhydrid in 3N-Natronlauge. Man rührt das Reaktionsgemisch 2 Stunden bei Raumtemperatur und filtriert vom ausgefallenen Quecksilber ab. Man giesst das Filtrat auf Eiswasser, extrahiert mit Aether, wäscht die organische Phase mit gesättigter Kochsalzlösung, trock-

net sie über Natriumsulfat und engt am Rotationseva- tor ein. Durch fraktionierte Destillation des erhält man das 2-(4-Phenoxyphenoxy)-cyclopentan- farbloses Oel vom $Kp_{0.001}$ 181-185°.

Das in diesem Beispiel verwendete Ausgangsmate- rial lässt sich z.B. wie folgt erhalten:

Zu einer Lösung von 18.6 g 4-Phenoxyphenol in ml Aceton gibt man 15,2 g wasserfreies Kaliumcarbonat und kocht das Reaktionsgemisch 1 Stunde am Rückfluss. Man fügt 15,7 g Cyclopent-2-enylbromid zu, kocht weitere 16 Stunden am Rückfluss, kühlt ab, filtriert und dampft das Filtrat Rotationsvaporisator ein. Das erhaltene Oel wird in Aether aufgenommen, die organische Phase viermal mit 10%-iger Kaliumhydroxydlösung und zweimal mit gesättigter Kochsalz- lösung gewaschen, über Natriumsulfat getrocknet und am Ro- tationsvaporisator eingeengt. Das erhaltene 3-(4-Phenoxy- phenoxy)-cyclopenten kann ohne weitere Reinigung verwendet werden.

Beispiel 7

Man rührt ein Gemisch aus 18,6 g 4-Phenoxyphenol, 12, 8g 1,2-Carbonyldioxycyclopentan und 4,6 g Triäthyl- niumjodid 7 Stunden bei 150°. Nach dem Abkühlen wird das tionsgemisch in Chloroform aufgenommen, die erhaltene Lö- sung mit Wasser und einmal mit gesättigter Kochsalzlösung gewaschen, über Natriumsulfat getrocknet und am Rotations- evaporator eingeengt. Durch fraktionierte Destillation des Rückstands erhält man 2-(4-Phenoxyphenoxy)-cyclopentan-1- ol als farbloses Oel vom $Kp_{0.001}$ 181-185°.

BAD ORIGINAL

**Beispiel 8**

Herstellung der Verbindung der Formel

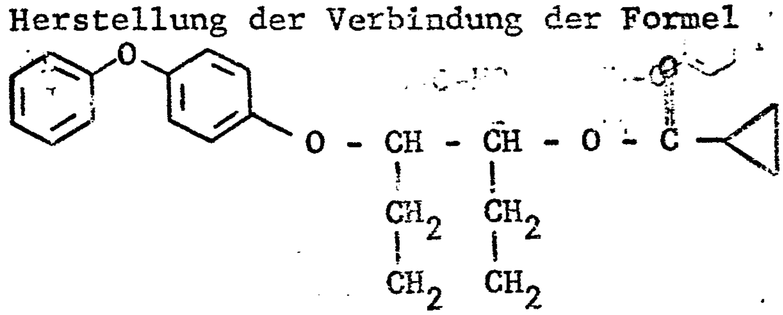

Zu einer Lösung von 28,3 g 2-(4-Phenoxy)-phenoxy-cyclohexanol in 150 ml wasserfreiem Benzol fügt man 12,1 g Triäthylamin hinzu und tropft anschliessend unter Rühren innerhalb einer Stunde bei 15-20° 11,5 g Cyclopropancarbonsäurechlorid zu. Das Reaktionsgemisch wird 24 Stunden bei Raumtemperatur weiter gerührt, hierauf nacheinander und wiederholt mit Wasser, verdünnter Salzsäure, 10%-iger Natriumcarbonatlösung und schliesslich mit gesättigter Kochsalzlösung neutral gewaschen. Die organische Phase wird abgetrennt, über Natriumsulfat getrocknet und im Vakuum vom Lösungsmittel befreit. Der ölige Rückstand wird an Kieselgel chromatographisch weiter gereinigt (Eluiermittel: Methylacetat-Hexan 1:4).

Man erhält die Verbindung der Formel IV mit einer Refraktion von $n_D^{20}$: 1,5542.

Auf analoge Weise werden auch die folgenden Verbindungen hergestellt:

physikalische Daten

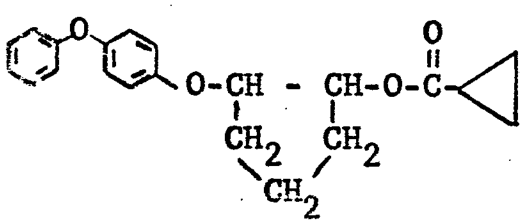

$n_D^{20}$ : 1,5590

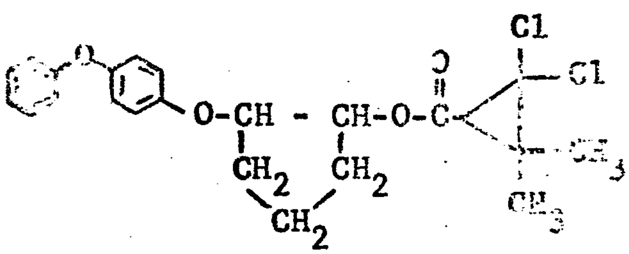

$n_D^{20}$ : 1,5574

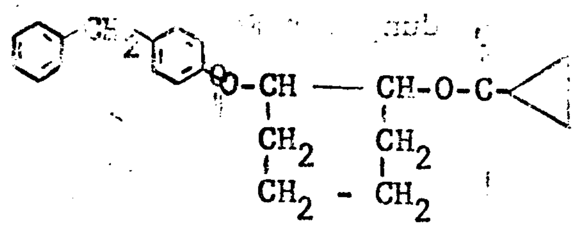

$n_D^{20}$ : 1,[illegible]

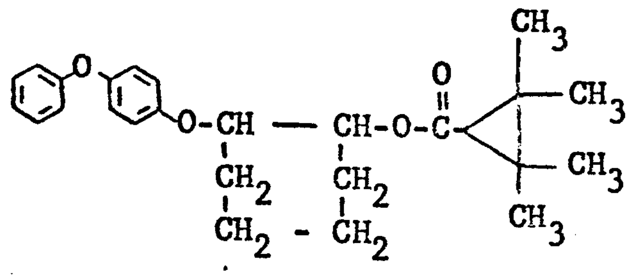

$n_D^{20}$ : 1,5421

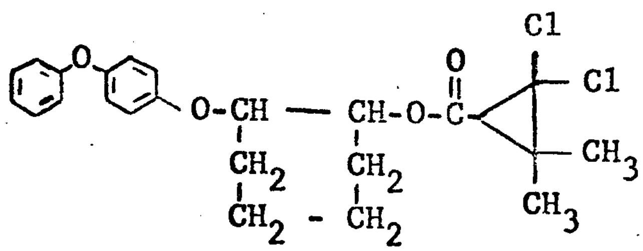

$n_D^{20}$ : 1,5552

## Patentansprüche

1. Verätherte Cycloalkanole der Formel

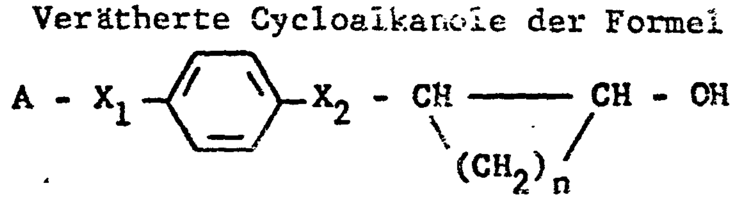

(I)

worin $X_1$ Sauerstoff, Schwefel oder Methylen, $X_2$ Sauerstoff oder Schwefel, A einen gegebenenfalls substituierten Phenylrest und n eine ganze Zahl von 1 bis und mit 10 darstellen.

2. Verätherte Cycloalkanole der Formel

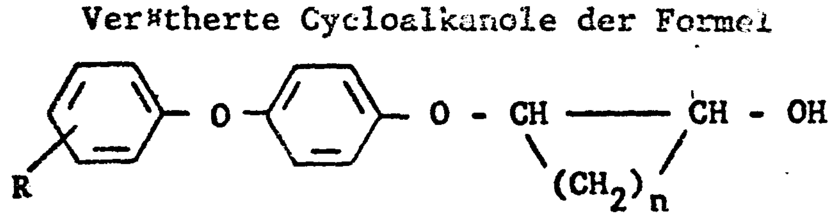

(II)

worin R Wasserstoff oder Chlor und n die ganzen Zahlen 3, 4 oder 5 darstellen.

3. 2-(4-Phenoxy-phenoxy)-cyclopentan-1-ol.

4. 2-[4-(4-Chlor-phenoxy)-phenoxy]-cyclopentan-1-ol.

5. 2-[4-(4-Chlor-phenoxy)-phenoxy]-cyclohexan-1-ol.

6. Verätherte Cycloalkanole der Formel

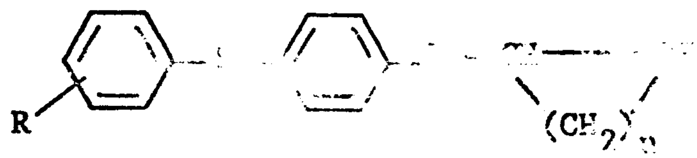

worin R Wasserstoff oder Chlor und n die ganzen Zahlen 3, 4 oder 5 darstellen.

7. 2-(4-Phenylmercaptophenoxy) cyclopentan-1-ol.

8. 2-(4-Phenylmercaptophenoxy)-cyclohexan-1-ol.

9. 2-[4-(4-Chlor-phenylmercapto)-phenoxy]-cyclopentan-1-ol.

10. 2-[4-(4-Chlor-phenylmercapto)-phenoxy]-cyclohexan-1-ol.

11. Verätherte Cycloalkanole der Formel

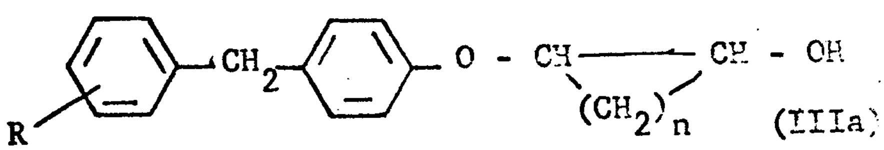

(IIIa)

worin R Wasserstoff oder Chlor und n die ganzen Zahlen 3, 4 oder 5 darstellen.

12. 2-(4-Benzylphenoxy)-cyclopenten-1-ol.

13. 2-(4-Benzylphenoxy)-cyclohexan-1-ol.

14. Pharmazeutische Präparate enthaltend eine der in den Ansprüche 1-13 beanspruchten Verbindungen zusammen mit einem Trägerstoff.

15. Die Verwendung der in den Ansprüchen 1-13 beanspruchten Verbindungen als Hypolipidaemika.

16. Verfahren zur Herstellung von verätherten Cycloalkanolen der Formel

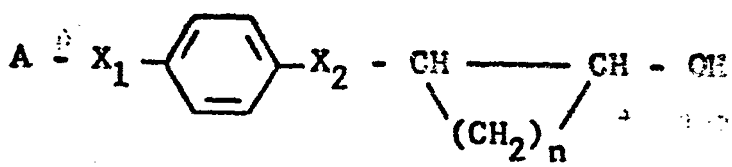

(I)

worin $X_1$ Sauerstoff, Schwefel oder Methylen, $X_2$ Sauerstoff oder Schwefel, A einen gegebenenfalls substituierten Phenylrest und n eine ganze Zahl von 1 bis und mit 10 darstellen, dadurch gekennzeichnet, dass man

a) eine Verbindung der Formel

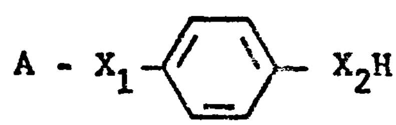

(IV)

oder ein Salz davon, mit einer Verbindung der Formel

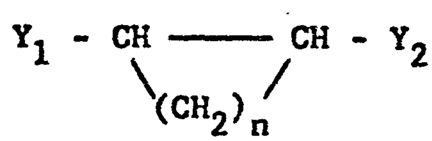

(V)

umsetzt, worin $Y_1$ eine freie oder reaktionsfähig veresterte Hydroxygruppe und $Y_2$ eine gegebenenfalls geschützte Hydroxygruppe oder $Y_1$ und $Y_2$ zusammen eine Epoxygruppe oder eine Carboxyldioxygruppe der Formel -OC(O)O- darstellen, und in einer erhaltenen Verbindung eine gegebenenfalls vorhandene Schutzgruppe abspaltet oder

b) in einer Verbindung der Formel

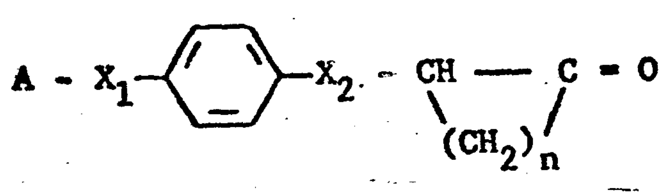

(VI)

die Ketogruppe in an sich bekannter Weise zur Carbinolgruppe reduziert, und, wenn erwünscht, ein erhältliches Isomerengemisch in die einzelnen Isomeren auftrennt.

17. Cyclopropancarbonsäureester der Formel

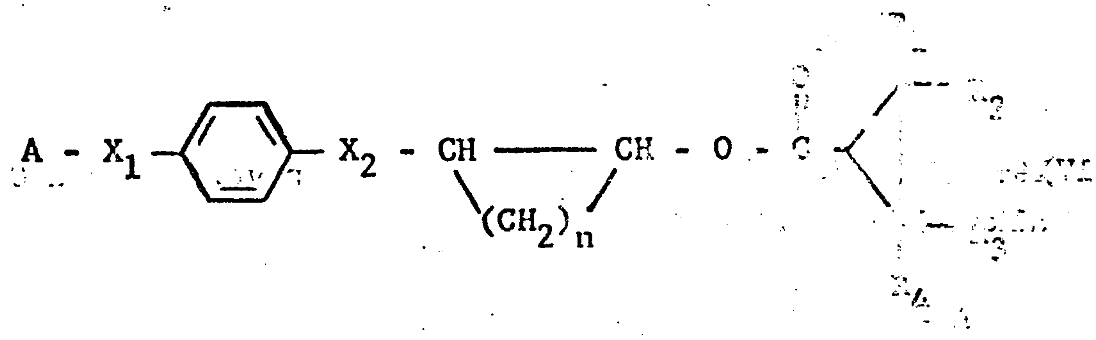

(VII)

worin $X_1$ Sauerstoff, Schwefel oder Methylen, $X_2$ Sauerstoff oder Schwefel, A einen gegebenenfalls substituierten Phenylrest, n eine ganze Zahl von 1 bis und mit 10 und $R_1$, $R_2$, $R_3$ und $R_4$ je Wasserstoff, Methyl oder Chlor bedeuten.

18. Cyclopropancarbonsäureester der in Anspruch 17 gegebenen Formel (VII), worin A Phenyl, $X_1$ und $X_2$ Sauerstoff und n, $R_1$, $R_2$, $R_3$ und $R_4$ die in Anspruch 17 gegebene Bedeutung besitzen.

19. Cyclopropancarbonsäureester der in Anspruch 17 gegebenen Formel (VII), worin A Phenyl, $X_1$ und $X_2$ Sauerstoff, $R_1$, $R_2$, $R_3$ und $R_4$ entweder Wasserstoff oder Methyl und n die oben gegebene Bedeutung besitzt.

20. Verwendung der in den Ansprüchen 17-19 beanspruchten Cyclopropancarbonsäureester als Insektizide.